# EUROPEAN PATENT APPLICATION

(11) **EP 0 003 529 A1**
(43) Date of publication of application: **22.08.1979**
(21) Application number: 79100209.0
(22) Date of filing: 24.01.1979
(51) Int. Cl.: A61K 39/00, C12K 5/00

(54) **Antigens from Trypanosoma cruzi, purification thereof, and their use in vaccines**

(30) Priority: 25.01.1978 GB 297578
(71) Applicant: THE WELLCOME FOUNDATION LIMITED, London NW1 2BP (GB)
(72) Inventor: McHardy, Nicholas, Beckenham Kent (GB); Thomas, Derek, West Wickham Kent (GB)
(74) Representative: Berg, Wilhelm, Dr.

(57) **Abstract**

An antigenic preparation contains antigens free from lipid which are isolated from the microsomal fraction of T.cruzi epimastigotes. Disruption of the epimastigotes followed by centifugation and separation of the microsomal fraction, followed by cleaving off the lipid moiety with a detergent yields the microsomal antigens. Their immunogenicity is enhanced by removal of material under 50 000 molecular weight, especially of material under 300 000 molecular weight. The preparation is incorporated into a vaccine. Two doses of the vaccine each containing the antigen from 10⁸-10⁹ epimastigotes, injected 14 days apart confer immunity to Chagas' Disease, which is uncurable by chemotherapy.

## Description

This invention relates to a vaccine against Chagas' disease. More particularly, this invention relates to a purified Trypanasoma cruzi antigen obtained from the cultured epimastigote stage of the parasite.

Trypanasomes are osmotrophic protozoa which are frequently parasitic in veritebrates, developing within cells and free in the blood-stream where they absorb nutrients from the infected host. Infoctions are readily transmitted via intermediate insect vectors. The American Trypanasoma cruzi causes Chagas' disoase which is endemic in several countries of Central and Scuth America, especially Mextico, Brazil. Chile, Argentina and Venezuela, as well as in parts of the southern United States. Commeon vertebrate hosts inelude man, household pets, for example dogs and cats; and wild mammals, for example bats, certain monkeys and oppossums. Because the organisms arc easily transmitted by the common reduyiid bugs, the incldence of human infections by virulent T_{.} cruzi is very high. The World Health Organisation estimated in 1970 (WHO Scientific Publication No.195) that at least seven million people in Latin America are infected with T cruzi, with another 35 million persons exposed to infections by this parasite.

Chagas' disease is especially dangerous because at the present time there are no satisfactiory prophylactic or curative therapeutic agents available, and because, as far as is known, once the disease is contracted by a patient, the individual remains infected until death. While T. cruzi is transmitted to man and animals naturally by blood sucking bugs, transmission by blood transfusion has become the second most important means of transmission in recent years.

A number of different morpholgieal stages of T. crui are known to exist depending on the evironment in which they occur. The morphological stages that occur in the body of the infected mammal are the trypomastigote and amatigote stages, When these forms are placed into in vitro axenic culture or are ingested by the insect vector. they may be induced into change into a third type called the epimasttigote. This latter stage is relatively easily grown and has been utilised for many years in the production of antigen for studies involving immunisation as well as for diagnostic procedures.

Because of the present lack of effective chemotherapeutic treatment for Cha gas' disease, several attempts have been made to develop vaccines effective for immunisation and diagnostic agents for the demonstration of protective antibodies. Vaccines and other antigenic preparations which have been used with the African Sleeping sickness-causing trypanasomes are unsuitable for use with the Ameuican T. cruzi which is a morphologically, biologically, patiiologically and immunologically distinct protozoan. Experimental vaccines against T. cruzi have previously been prepared from the epimnastigote stage present in axenic culture and in the insect vector, or from immunologically related protozoa.

Antigens have been prepared for immunological studies of Chagas' disease by several methods. The mechanical rupture of T. Cruzi organidms has been accinokusaed by freeze-thawing, trituration with or without beads, and sonicaticn, and evidence that mice are partially protected from acute trypanasomiasis by the injection of homogenatcs, obtaiaed by these phtsical methods has been reported (Johnsen P., and Neal, R.A., Nature. 1963, 200, 83; Govle F.C. et, al., J.Parasito 1964, 50 (Suhpl.e.), 19; Sernecu. H. et al. Nature, 1956. 209,309-310; and Seneca. H. 8th International Congress of Internal Medicine, 1965, page J56, Buenos Aires, Argentina). However, generally a decrease in paxasitaemia but little increase in survival was observed.

It has also been shown that a homegenate of epimastigotes of T.cruzi prepared by 140 atm. of pressure in a nitrogen atmosphere with precise temperature control showed enhanced stability for 8 hours and protective activity in mice against subsequent challenge with . lethal doses of the parasite. The homogenate prepared at this pressure preserved the subcellular structures intact and permitted their isslation (Segura, E.L. et al. J.Protozool, 21, 571 -574). Subsequent to this work, Gonzalez Cappa S.M. ct al (J.Parasitology 1976, 62,130) and Segura, E.L. et al. (Ibid.131) disrupted epimastigotes of T.cruzi by nitrogen cavitation and separated fractions of the homogenate by centrifugation. They discovered that protective activity was closely related with the flagellar fraction since immunisation of mice by the intraperitoneal route with this fraction gave 90% survival. The so-called membrane fraction showed some activity, but was not as efficient as the flagellar fraction, whilst no activity was shown by cell sap, and protection was very poor for the microsomal fraction.

It has now been found that a most effective microsomal antigen against T. cruzi infection may be obtained by a disruption of the eplmastigots stage of the organism and by a solubilisation of the fraction witch is enriched in microsomal antigen. Such solubilisation involves a treatment which cleaves off and removes the lipid moiety of such antigens.

According to the present invention in one aspect therefore there is provided an antigen preparation obtained from the epimastigote stage of T. cruzi, said antigen comprising the microsomal antigen in a sub- stantiallylipid free form. In a particular aspect such preparation is substantially free from material of epimastigote origin having a molecular weight of less about 50 000.

The epimastigote stage of T. cruzi for use in the present invention may conveniently be groun in axenic culture, for example in Bone and Parent's medium +5% rabbit serum or stearate (Bime, G.J. and Parent, J.Gen. Mierobiol. 1963, 31 , 261.. 266). The organisms are allowed to grow at 24°Cto 30°C preferably 26°C for up to 9 days after which time they arc harvested and washed, for example, in 0.15M saline soiution or phosphate buffored saljae. The organiams obtained in this manner are then subjected to a process for the separation of microsomal antigens from other antigens such as for instance those associated with the flagellae, and the solubilisation of the same by chemical means. According to another aspect there is provided a process of preparing the said antigen preparation, which comprises the steps of
(a) disrupting the epimastigote growth stage of T. cruzi organisms;
(b) separating a fraction containing a relatively increased proportion of microsomal antigens; and
(c) solubilising the antigen fraction so obtained by the use of agents capable of cleaving off the lipid moiety of the microsomal antigens.

In a particular aspect, such a process includes a removal of material having a molecular weight of less than about 50,000, for instance by means of molecular sieving. Furthermore the process may also include an additional preliminary fractionation of the antigen material for instance by the use of centrifugation in a density gradient and a selection of the fraction with the highest immunogenicity i.e. the peak fraction showing the highest purity in respect of protective antigens.

After harvesting and washing the organisms from a T. cruzi culture, the cells are disrupted by known techniques, such as freeze-thawing or pressure cavitation, but according to the present invention it is preferred to use ultrasonication of the cell suspension to destroy the cellular and subcellular structure, in order to release cellular meterial therefrom, particularly on small scale. The disruption of the epimastigote growth stage is done not only to release cellular material but also to ensure that no viable organisms remain. In addition, to make quite certain that no live organisms arc left after ultrasonication, it is preferable that a killing agent, for example formalin, as added to the ultrasonicate to a final concentration in the range of 0.01% to 1.00% preferably 0.1%.

For a small volume of material, up to SO ml., ultrasonication. is conveniently performed by means of ultrasenic waves having a wavelength in the range of 15 to 25 Kliz, and amplitude of 5 to 20, preferably 12 µm, peak to peak, for a period of about one minute. One commercial scale, other methods of disruption may well be more advantageous.

After disruption, the resulting homogonate is fractionated for instance by lifferential centrifugatio. Firstly, the hnmogenate may be centrifuged at 9000 to 15 000 g., preferably 12 000 g. for a time sufficent to allow complete deposition of unwanted material from the resulting supernatant liquid. Normally this can take time of the order of 10 minutes. Subsequent to this centrifugation, the supernatant layer is removed and further centrifuged at 90 000 to 120 000 g., preferably about 102 000g. for approximately one hour to achieve a deposition of particulate material, representing the fraction containing microsomal antigens.

The material from the separation step is either dlrectly subjected to solubilisation or else is subjected to further fractionation such as centrifugation on a gradient before solubilisation. The incorporation of such an additional purification step has the advantage that a more potent antigen results although the overall yield of the material may then be lower.

Solul ilisation for the purposes of the present invention involves the use of an agent which increases the waten solubility of the microsomal antigen by means of splitting off its lipid component, without damaging its immonogenicity. Such agents include detergents, including both the non-ionic and ionic types. The former may be representei by agents like Triton or Nonidct, (Registered Trade Marks) (Manufactured by Rohm and Haas and by Shell, rcspectivelly).
the latter includes deoxycholate, and ammonix L0, but a solution of sodium dodecyl sulphate is most preferred.

Pharmaceutically acceptable detergents are recommended for the purpose, although the free remnants of the agent are normally separated from the antigen whenever molecular sieving succeeds solubilisation. The concentration of the detergent is preferably from 0.1% to 2.0% usually around 0.5%.

The quality of the antigen can be further improved by incorporating a fractionation step in the process before solubilisation, for instance by zonal gradient fractionation or by centrifugation on a sugar gradient. Conveniently the material deposited after the centrifugation at high speed is resuspended in a sugar solution, for example a sucrose solution, having a concentration in the range of 0.05M to 0.5M, preferably 0.1M to 0.4M, most preferably 0.25M, which liquid suspension is then layered onto a linear sugar, for example sucrose gradient from 10% to 50% and centrifuged at 50 000 to 80 000 g. for approximately fifteen hours. After this time material at the 30% to 50% level, preferably around the 36 level,is collected.

After solubilising the antigenic fraction so obtained, the molecular sieving is performed by metheds well known in the art, for instance by employing zeolites which are natural hydrous metal alumino-silicates containing regular channels of molecular dimensions throughout their very open crystal structures.Alternatively, cross-linked dextrans may be used. By selecting appropriate agents for the purpose, material having a molecular weight of greater than 50 000 can be retained. This portion has been found to be highly immunogenic in tests carried out in vivo.

The liquid passing through the zeolite is discarded and the retained material is eluted by known methods to provide the desired purified fraction. The antigens obtained in this manner include those which have either a molecular weight higher than 50 000 or comprise aggregates of smaller components. The latter type of material can provide improved immunogenicity for the preparation.

An even more immunogenic antigen may be prepared by further molecular sieving. Although material which is free of material of cpimastigole origin having a molecular weight of less than 50 000 already manifests improved antigenicity, it hes been found that further fractionation to remove material with a molecular weight of less than 300 000, provides a further increase in antigenicity. The removal of material having a M. Wt. of < 300 000 can either be performed subsequent to the first molecular sieving operation wherein material having a M.Wt. of < 50 000 has been removed, or else be done directly after the solubilisation with a molecular sieve of appropriate capability.

The modified microsomal antigens of epimastigote origin prepared according to the present invention may have additional advantages in view of the preferential removal of antigens associated with the flagellae of the organisms. Since the latter type of antigen is likely to give rise to andesirable autoimmunogenic side effects, the antigens produced in the manner of the present invention would reduce: such risks.

The antigen as herinbefore defined may be formulated intc vaccines suitable for administration to susceptible hosts, such as mammals, including humans at risk to be infected with T.cruzi. This may conveniently be done by suspending the antigen in a pharmaceutically acceptable carrier, for example phosphate buffered saline.

According to the present invention in a further aspect, there is provided a vaccine for immunisation against T.cruzi, which comprises an antigen as hereinbefore defined, in a pharmaceutically acceptable carrier.

Preferably the antigen is combined with an adjuvant for stimulating the immune response of the infected host and therefore increasing the effectiveness of the vaccine. Suitable adjuvants for use in the present invention include Freund's complete adjuvant, and more particularly saponin for animals, and Corynebacterium parvum (cuparvax) or aluminium hydroxide, for humans.

After formulation the vaccine is conveniently incorporated into a sterile container, which is then sealed and stored at a low temperature, for example 4°C, or may be freeze-dried.

The vaccines of the present invention are desirably administered by subcutaneous or intrsmuscular injection although the intravenous injection route may be employed. The dose may be administered to hosts as one unit, or as a multiplicity of sub-dcses over a pcried cf time. A more suitable schedule is to administer two doses of the vaccine with 7 to 56 preferably 14 day interval between them. If jonger protection is required then booster dose shuuld be given at approximately yearly intervals. Fach dose of an immunising injection desirably contains the product of 10⁷ to 10¹⁰ epimastigotes, more preferably 10⁸ to 10⁹ epimastigotes.

The present invention in a further aspect provides a method for the prevention of T cruzi infection in susceptible hosts, comprising the administration of an effective anti-protczoal dose of a vaccine, as hereinbefore defined,to the host.

The following Example serves to illustrate the present invention but is not intended to limit it in any way.

### EXAMPLE 1

A culrure of epimastigotes of T. cruzi was grown in Bené and Patent's liquid medium (Boné, G.J., et al., J. Gen Micrebiol., 1963, 31, 261-266) with 5% rabbit serum added. The cultures were established with 1 x 10⁷ epimnstigotes/ml. and incubated at 26 C for 4-7 days, in glass bottles, containing 1000 ml. of the medium, with gentle agitation At the end of the culture period, the medium contained 204 x 10⁸ epimastigotcs/ml. These were separated from the supernatent by
centrifugation at 2000 g for 15 minutes, then washed twice with phosphate-buffered saline. The deposit was resuspended in phosphate- buffered saline (4 x 10⁹ organisms/ml)and then subjected to ultrasonication at 20 KHz frequency, and 12 µm amplitude for about 1 minute.

The disrupted cultured epimastigotes of T.cruzi were centrifuged at 12 000 g. for 10 minutes and the deposit was discarded. The resulting supernatant was then centrifuged at 102 000 g. for a further 10 minutes. The deposited material was subsequently resuspended in 0.25M sucrose solution and layered onto the surface of a linear sucrose gradient, from 10-50%, and centrifuged at 64 000g. for 14 hours. The material at the 3% level was collected and a sample was examined using an electron microscope to confirm the ultra-structure of the collected fraction which appeared to be 'microsomal'.

The immuncgenicity of a sample of this fraction was tested before being subjected to molecular sieving according to the following procedure.

Gromps of 10 mice were immunised with whole ultrasonicate or the fraction prepared as above with sapcnin (50 µg) as adjuvant. Each vaccine dose contained the product of 10⁸ epimastigotes. As controls, one group of mice was hept unvaccinatcd and one group received sapenin only.

Two doses of vaccine were injected subcutaneously. with an interval of 14 days, and the mice were challenged 14 days after the second immunising dose by the subcutaneous injection of 2 x 10⁴ homologous blood trypomastigotes. The following results were obtained:-

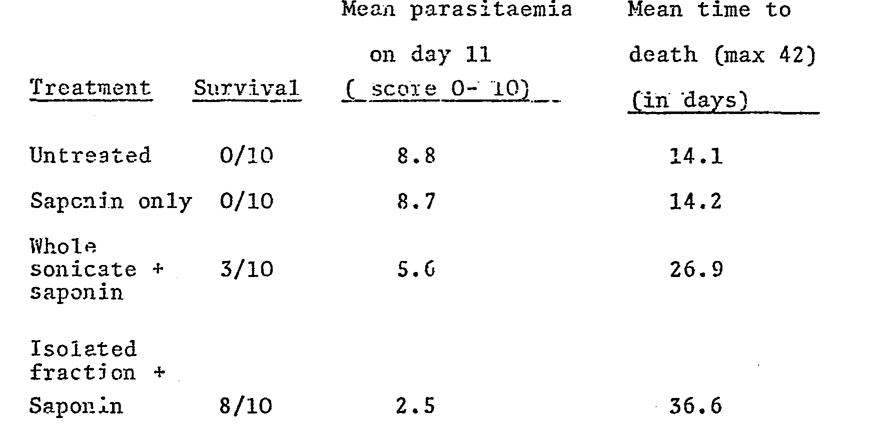

The fraction isolated as above was solubilised in an aqueous 0.5% solution of sodium dodecyl sulphate and then subfractionated by moleculor sieving to yield four fractions of different molecular weights:-

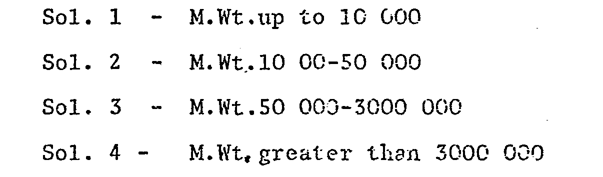

The immunogenicity of each of the above four sub. fractions was tested according to the following procedure.

Group of 10 male CD-1 mice were given two subcutaneous immunising injections with an interval of 14 days. Each antigen dose contained the product of 10⁸ epimastigotes. Saponin was used as adjuvant (50 µg/dose). The mice were challenged 14 days after the second immunising dose by the subcutaneous injection of 2 x 10⁴ homologous blood trypomastigotes. The following results were obtained:-

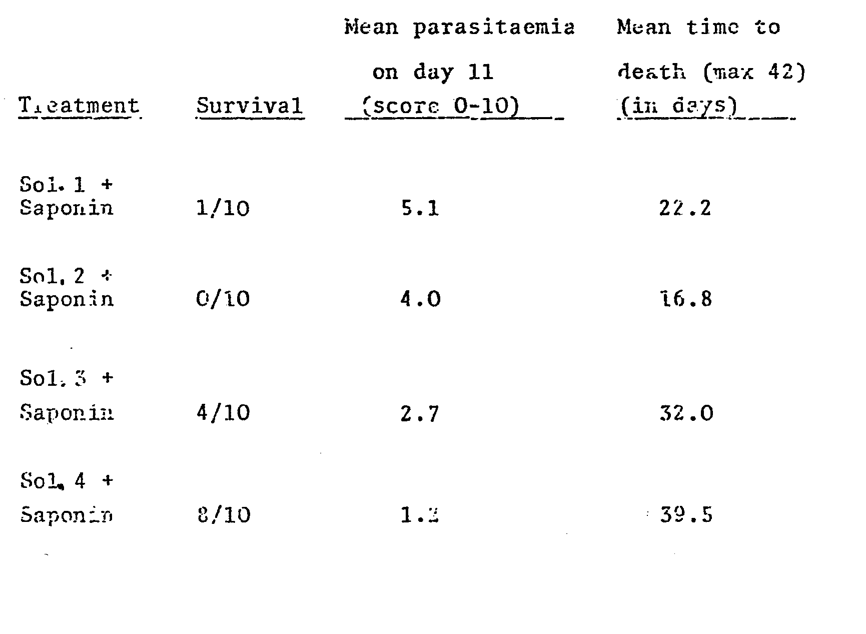

## Claims

1. An antigen preparation obtained from the epimastigote stage of T. cruzi characterised in that the preparation comprises microsomal antigen in a substantially lipid- free form.

2. An antigen preparation as claimed in claim 1 wherein the antigen substantially free of material of melecular weight below 50 000,

3. An antigen preparation as claimed in claim 1 or claim 2 wherein the antigen is substantially free of material of molecular weight below 300 000.

4. A process for production of the antigen characterised by the three steps of
a) disrupting the epimastigote growth stage of T.cruzi organisms;
b) separating a fraction containing a relatively increased proportion of microsomal antigen; and
c) solubilising to antigen fraction so obtained by the use of agents capable of cleaving off the lipid moiety of the microsomal antigens.

5. A process as claimed in claim 4 wherein a killing agent is added to the disrupted epimastigotes.

6. A process as claimed in claim 4 or claim 5 wherein the seiubi lising agent is a pharmaceutically acceptable detergent.

7. A process as claimed in anyone of claims 4 to 6 comprising an additional fractionation step and the selection of the fraction with highest immunogenicity, before step (c).

8. A process as claimed in any one of claims 4 to 7 comprising the additional stop of removal of material having a molecular weight of less than 50 000

9. A process as claimed in any one of claims 4 to 8 comprising the additional step of removal of material having a molecular weight less than 300 000.

10. A vaccine for inducing immunity againt T.cruzi characterised in that it comprises an antigen preparation as descrihed in any one of claims 1 to 3.

11. A vaccine as claimed in claim in 10 which also comprises an sdjuvant.
